# NEUE EUROPÄISCHE PATENTSCHRIFT

(11) **EP 0 753 545 B2**
(45) Veröffentlichungstag und Bekanntmachung des Hinweises auf die Entscheidung über den Einspruch: **31.05.2006**
(45) Hinweis auf die Patenterteilung: 02.10.2002
(21) Anmeldenummer: 96110783.6
(22) Anmeldetag: 04.07.1996
(51) Int. Cl.: C09C 1/00, C09D 7/12, C09D 11/00, C08K 3/00, C03C 4/02, C04B 33/14, A61K 8/19

(54) **Goniochromatische Glanzpigmente auf der Basis transparenter, nichtmetallischer, plättchenförmiger Substrate**
Goniochromatic brilliant pigments based on transparent non-metallic platy substrates
Pigments brillants goniochromatiques à base de substrats en plaquettes transparentes et non-métalliques

(30) Priorität: 13.07.1995 DE 19525503
(43) Veröffentlichungstag der Anmeldung: 15.01.1997
(73) Patentinhaber: BASF Aktiengesellschaft, 67056 Ludwigshafen (DE)
(72) Erfinder: Schmid, Raimund, Dr., 67435 Neustadt (DE); Mronga, Norbert, Dr., 69221 Dossenheim (DE)

(56) Entgegenhaltungen:
- EP-A- 0 589 681
- EP-A- 0 690 105
- EP-B- 0 373 575
- EP-B- 0 708 155
- WO-A-94/01498
- DE-A- 2 106 613
- DE-A- 2 429 762
- DE-A- 3 528 256
- DE-A- 4 319 669
- US-A- 4 017 326

## Beschreibung

Die vorllegende Erfindung betrifft neue goniochromatische Glanzpigmente auf der Basis von mehrfach beschichteten, hochbrechenden, für sichtbares Licht zumindest teilweise durchlässigen, nichtmetallischen, plättchenförmigen Substraten, die mindestens ein Schichtpaket aus
A) einer farblosen Beschichtung mit einem Brechungsindex n ≤ 1,8 und
B) einer reflektierenden, selektiv oder nichtselektiv absorbierenden, für sichtbares Licht zumindest teilweise durchlässigen Beschichtung sowie gewünschtenfalls zusätzlich
C) eine äuBere Schutzschicht
aufweisen, wobei die Schichtdicke der Schicht (A) 100 bis 600 nm beträgt, wenn die Glanzpigmente ein Schichtpaket (A) + (B) aufweisen, und 50 bis 300 nm beträgt, wenn die Glanzpigmente mehrere Schichtpakete (A) + (B) aufweisen, und ihre Verwendung zum Einfärben von Lacken, Druckfarben, Tinten, Kunststoffen, Gläsern, keramischen Produkten und Zubereitungen der dekorativen Kosmetik.

Glanz- oder Effektpigmente werden in vielen Bereichen derTechnik eingesetzt, beispielsweise In Automobillacken, In der dekorativen Beschichtung, der Kunststoffeinfärbung, in Anstrich-, Druck-, Insbesondere Sicherheitsdruckfarben sowie in der Kosmetik.

lhre optische Wirkung beruht auf der gerichteten Reflexion von Licht an überwiegend flächig ausgebildeten, zueinander parallel ausgerichteten, metallischen oder stark llchtbrechenden Pigmentteilchen. Je nach Zusammensetzung der PigmentpläfthL-n erzeugen Interferenz-, Reflexions- und Absorptionsphänomene winkelabhängige Farb- und Helligkeitseindrücke.

Aufgrund ihrer nicht kopierbaren optischen Effekte gewinnen diese Pigmente zunehmende Bedeutung für die Herstellung von fälschungssicheren Wertschriften, wie Geldscheinen, Schecks; Scheckkarten, Kreditkarten, Steuermarken, Briefmarken, Bahn- und Flugtickets,Telefonkarten, Lotterielosen, Geschenkzertifikaten, Ausweisen und identifikationskarten.

Kennzeichnungen, die mit den Effektpigmenten angefertigt wurden, und das Fehlen dieser Kennzeichnungen oder ihre Veränderung, beispielsweise in einer Farbkopie (Verschwinden von Farbflops und Glanzeffekten), sind ohne Hilfsmittel mit bloßem Auge sicher erkennbar und ermöglichen so eine leichte Unterscheidung des Originals von der Kopie.

Goniachromatische Glanzpigmente, die einen winkelabhängigen Farbwechsel zwischen mehreren intensiven Interferenzfarben zeigen und aufgrund lhres Farbenspiels von besonderem Interesse sind, sind bislang nur auf Basis mehrfach beschichteter, plättchenförmiger, metallischer Substrate bekannt.

In den US-A-3 438 796 und 5 135 812 werden z.B. metallische Glanzpigmente beschrieben, die einen zentralen opaken Aluminiumfilm aufweisen, der beidseitig alternierend mit dielektrischen, niedrigbrechenden Filmen (Siliciumdioxid, Magnesiumfluorid) und transparenten Metallfilmen (Aluminium, Chrom) beschichtet ist. Aufgrund Ihrer zudem sehr aufwendigen Herstellung (abwechselndes Bedampfen einer Substratfolle im Hochvakuum mit den unterschiedlichen Filmmaterialien, Entfernen der Folie von dem aufgedampften mehrschichtigen Film und dessen Zerkleinerung auf Pigmentteilchengröße) ist der zentrale Metallfilm dieser Pigmente nur an der Plättchenober- und -unterseite beschichtet.

Aus der nicht vorveröffentlichten DE-A-44 05 492, den älteren deutschen Patentanmeldungen 19516181.5 und 19515988.8 und der nicht vorveröffentlichten DE-A-44 37 753 sind gonlochromatische Glanzpigmente bekannt, die durch Beschichtung von Metallplättchen (insbesondere Aluminiumplättchen) über CVD-Verfahren (chemical vapor deposition) oder naßchemisch mit niedrigbrechenden Metalloxidschichten (insbesondere SiO₂) und nichtselektiv absorbierenden, Metalle, Metalloxide und oder Metallsulfide enthaltenden Schichten oder selektiv absorbierenden, hochbrechenden Metalloxidschichten hergestellt werden.

Die Glanzpigmente auf metallischer Basis welsen zwar gute Anwendungseigenschaften, u.a. ein gutes Deckvermögen, auf, jedoch resultiert bel der Applikation z.B. im Lack ein "harter" metallischer Glanz, der mitunter nicht erwünscht ist.

Glanzpigmente auf Basis transparenter, plättchenförmiger Substrate, die diesen harten metallischen Glanz nicht zeigen, werden z.B. in der WO-A-93/12182 beschrieben; die mit einer hochbrechenden Metalloxidschicht (insbesondere Titandioxid) und einer nichtselektiv absorbierenden Schicht belegte Glimmerplättchen betrifft. Diese Pigmente zeigen abhängig von der TiO₂₋Schichtdicke in der Aufsicht eine bestimmte Interferenzfarbe, die mit schräger werdendem Blickwinkel zunehmend schwächer wird und schließlich nach grau bzw. schwarz abkippt. Der Interferenzfarbton ändert sich hierbei nicht, es nimmt lediglich die Farbintensität (Farbsättigung) ab.

Glanzpigmente auf der Basis von Siliciumdioxidpiättchen, die in aufwendiger Weise durch Aufbringen einer Wasserglaslösung auf ein Substratband, Gelierung, Trocknung, Ablösung, Auswaschen der Salze und Zerkleinerung des "SiO₂-Films" hergestellt werden, sind aus der WO-A-93/8237 bekannt. Durch Einfärbung des SiO₂-Films mit organischen oder anorganischen Pigmenten und Beschichtung der gefärbten SiO₂-Plättchen mit SnO₂-haltigem Titandioxid werden Pigmente erhalten, die winkelabhängig entweder die Interferenzfarbe oder die Körperfarbe des Pigments zeigen.

SchlieBlich werden in der JP-A-93206/1992 Glanzpigmente auf der Basis von Glasflakes, die mit einer opaken Metallschicht und alternierenden SiO₂- und TiO₂-Schichten belegt sind, beschrieben, die den metallischen Glanzpigmenten ähneln.

Der Erfindung lag daher die Aufgabe zugrunde, weitere goniochromatische Glanzpigmente bereitzustellen, die sich durch vorteilhafte Anwendungseigenschaften auszeichnen und auf wirtschaftliche Weise hergestellt werden können.

Demgemäß wurden die eingangs definierten goniochromatischen Glanzpigmente und ihre Verwendung zur Einfärbung von Lacken, Druckfarben, Tinten, Kunststoffen, Gläsern, keramischen Produkten und Zubereitungen der dekorativen Kosmetik gefunden.

Die erfindungsgemäßen goniochromatischen Glanzpigmente weisen hochbrechende, für sichtbares Licht zumindest teilweise durchlässige, nichtmetallische, plättchenförmige Substrate auf.

Unter "für sichtbares Licht zumindest teilweise durchlässig" ist dabei zu verstehen, daß in der Regel mindestens 10 %, bevorzugt mindestens 30 %, des auftreffenden Lichts vom Substrat durchgelassen werden.

Als Substratmaterial sind dabei (semi)transparente Materialien geeignet, die "von sich aus" hochbrechend sind, d.h., einen Brechungsindex von in der Regel ≥ 2, vorzugsweise ≥ 2,4 haben, oder die "von sich aus" nur niedrigbrechend sind und mit einer hochbrechenden, lichtdurchlässigen Beschichtung versehen sind.

Beispiele für besonders geeignete von sich aus hochbrechende Materialien sind vor allem plättchenförmige Eisenoxide, bevorzugt plättchenförmiges Eisen(III)oxid α-Fe₂O₃, das mit Silicium (EP-A-14 382), Aluminium (EP-A-68 311) oder Aluminium und Mangan (EP-A-265 820) dotiert ist sowie plättchenförmiges Bismutoxychlorid BiOCI (EP-A-315 849). Prinzipiell sind auch plättchenförmiges Titandioxid und Zirkondioxid geeignet, diese Materialien können jedoch nur in aufwendiger Weise hergestellt werden (US-A-4168 986).

Beispiele für besonders geeignete, von sich aus nur niedrigbrechende, aber mit hochbrechendem Material beschichteten Materialien sind vor allem silikatische Plättchen, die mit einer hochbrechenden Metalloxidschicht belegt sind. Als silikatische Plättchen kommen dabei insbesondere helle bzw. weiße Glimmer in Betracht, wobei Schuppen von vorzugsweise naß vermahlenem Muskovit besonders bevorzugt sind. Selbstverständlich können auch andere natürliche Glimmer wie Phlogopit und Biotit, künstliche Glimmer, Talk- und Glasschuppen verwendet werden.

Die Metalloxidbeschichtung der silikatischen Plättchen kann aus farblosen hochbrechenden Metalloxiden wie Titan-, Zirkon-, Zink-, Zinnoxid und Bismutoxychlorid und absorbierenden hochbrechenden Metalloxiden wie Eisen- und Chromoxiden, Ilmenit oder auch Gemischen dieser Oxide aufgebaut sein. Aluminium- und Siliciumoxid können ebenfalls, jedoch nur in untergeordneter Menge, enthalten sein.

Besonders bevorzugte Substratmaterialien sind Glimmerplättchen, die eine im wesentlichen aus Titandioxid bestehende Oxidbeschichtung aufweisen, welche nur geringe Mengen (im allgemeinen < 5 Gew.-%) weiterer, vorzugsweise farbloser, Metalloxide enthält. Derartige Pigmente sind allgemein bekannt und im Handel unter den Bezeichnungen Iriodin® (Merck, Darmstadt), Flonac® (Kemira Oy, Pori) oder Mearlin® (Mearl Corporation, New York) erhältlich.

Die Dicke der TiO₂-Schicht (geometrische Schichtdicke) beträgt üblicherweise 10 bis 300 nm, vorzugsweise 20 bis 200 nm. Besonders vorteilhaft können auch Glimmerpigmente mit nur dünnen TiO₂-Beschichtungen (etwa 20 bis 40 nm) als Substrate verwendet werden.

Von besonderem Interesse als Substratmaterial sind auch titandioxidbeschichtete Glimmerpigmente, deren TiO₂-Beschichtung teilweise reduziert ist und die neben unverändertem TiO₂ reduzierte Titanspezies mit Oxidationszahlen <4 bis 2 (niedere Oxide wie Ti₃O₅, Ti₂O₃ bis zu TiO, Titanoxynitride sowie Titannitrid) enthält. Die reduzierten Pigmente sind farbintensiver als die unreduzierten, TiO₂-belegten Pigmente, und ihre Körperfarbe verschiebt sich mit zunehmendem Reduktionsgrad in Richtung der Absorptionsfarbe der Reduktionsprodukte des Titans, also in den blauen bis violetten Farbtonbereich. lhre Herstellung kann bekanntermaßen durch Reduktion mit Ammoniak, Wasserstoff sowie auch Kohlenwasserstoffen und Kohlenwasserstoff/Ammoniak-Gemischen (vgl. EP-A-332 071 und die älteren deutschen Patentanmeldungen 1951696.8 und 19511697.6 sowie den dort beschriebenen Stand der Technik) erfolgen, wobei die in Gegenwart von Kohlenwasserstoffen reduzierten Pigmente in der Regel auch Kohlenstoff enthalten.

Die Größe der Substratteilchen ist bei den erfindungsgemäßen Glanzpigmenten an sich nicht kritisch und kann .auf den jeweiligen Anwendungszweck abgestimmt werden. In der Regel haben die plättchenförmigen Teilchen mittlere größte Durchmesser von etwa 1 bis 200 µm, insbesondere etwa 5 bis 100 µm, und Dicken von etwa 0,1 bis 1 µm, insbesondere um etwa 0,3 µm. Ihre spezifische freie Oberfläche (BET) liegt üblicherweise bei 1 bis 15 m²/g, insbesondere 1 bis 12 m²/g.

Die erfindungsgemäßen Glanzpigmente weisen eine farblose, niedrigbrechende Beschichtung (A) in Kombination mit einer reflektierenden Beschichtung (B) auf, die selektiv absorbierend oder nichtselektiv absorbierend sein kann, für sichtbares Licht aber in jedem Fall zumindest teilweise durchlässig sein soll. Sie können mehrere, gleiche oder verschiedene Kombinationen (Schichtpakete) (A) + (B) enthalten, bevorzugt ist aber die Belegung mit nur einem Schichtpaket (A)+ (B). Zusätzlich kann noch zum Schutz der darunter liegenden Schicht (B) eine äußere Schicht (C) aufgebracht sein.

Die niedrigbrechende Beschichtung (A) hat in der Regel einen Brechungsindex n≤1,8, vorzugsweise ≤1,6.

Als Schichtmaterial (A) eignen sich alle niedrigbrechenden farblosen Substanzen, die filmartig und dauerhaft auf die Substratteilchen aufgebracht werden können.

Besonders geeignet sind z.B. neben Magnesiumfluorid und Aluminiumphosphat vor allem Metalloxide wie Siliciumoxid, Siliclumoxidhydrat, Aluminiumoxid, Aluminiumoxidhydrat und deren Mischungen, wobei Siliciumoxid(hydrat) bevorzugt ist.

Da die Schicht (A) im wesentlichen die Interferenzfarben der erfindungsgemäßen Pigmente bestimmt, hat sie Glanzpigmente, die nur ein Schichtpaket (A) + (B) aufweisen, eine Schichtdicke von 100 bis 600 nm. Sind mehrere (z.B. 2, 3 oder 4) Schichtpakete (A) + (B) vorhanden, dann liegt die Schichtdicke von (A) bei 50 bis 300 nm.

Für die refektierende Beschichtung (B) eignen sich sowohl hochbrechende, selektiv oder nichtselektiv absorbierende Substanzen als auch niedrigbrechende, aber eine hohe Absorptionskonstante aufwelsende, nichtselektiv absorbierende Substanzen, die selbstverständlich auch filmartig und dauerhaft abscheidbar sein müssen.

Als hochbrechende, für die Beschichtung (B) geeignete Materialien sind z.B. nichtselektiv absorbierende Materialien wie Metalle, Metalloxide, Metallsulfide und deren Mischungen, die auch selektiv absorbierende Metalloxide in untergeordneter Menge enthalten können, und selektiv absorbierende Materialien wie insbesondere Metalloxide zu nennen, die in der Regel jeweils einen Brechungsindex n ≥2,0, vorzugsweise n ≥ 2,4, haben.

Im einzelnen seien als für die Beschichtung (B) geeignete, nichtselektiv absorbierende hochbrechende Materialien beispielsweise genannt:
- Metalle, die durch Gasphasenzersetzung flüchtiger Metallverbindungen aufgebracht werden können, wie besonders bevorzugt Molybdän, bevorzugt Eisen, Wolfram und Chrom, auch Cobalt und Nickel, sowie Gemische dieser Metalle; Metalle, die naßchemisch durch Reduktion aus Metallsalzlösungen abgeschieden werden können, wie Silber, Kupfer, Gold, Palladium und Platin sowie auch Cobalt und Nickel und Legierungen wie NIP, NIB, NiCo, NiWP, CoP und AgAu;
- Metalloxide wie bevorzugt Magnetit Fe₃O₄, Cobaltoxid (CoO, Co₃O₄) und Vanadiumoxid (VO₂, V₂O₃) sowie auch Mischungen dieser Oxide mit den Metallen, wie insbesondere Magnetit und Eisen;
- Metallsulfide wie besonders bevorzugt Molybdänsulfid, bevorzugt Eisensulfid, Wolframsulfid und Chromsulfid, auch Cobaltsulfid und Nickelsulfid sowie Gemische dieser Sulfide wie MoS₂/WS₂ und vor allem auch Gemische dieser Sulfide mit dem jewelligen Metall, wie insbesondere MoS₂ und Molybdän, und Gemische mit Oxiden des jeweiligen Metalls, wie MoS₂ und Molybdänoxide.

Als nichtselektiv absorbierende hochbrechende Beschichtung (B) eignen sich z.B. auch Schichten farbloser hochbrechender Materialien wie Zirkondloxid und insbesondere Titandioxid, in die nichtselektiv absorbierendes (schwarzes) Material (z.B. Kohlenstoff) eingebaut ist oder die mit diesem Material beschichtet sind (EP-A-499 864).

Als Beispiele für selektiv absorbierende hochbrechende Schichtmaterialien (B) sind insbesondere farbige Oxide wie bevorzugt Eisen(III)oxid (α-und γ-Fe₂O₃, rot), Chrom(III)oxid (grün), Titan(III)oxid (Ti₂O₃, blau) und auch Vanadiumpentoxid (orange) sowie farbige Nitride wie bevorzugt Titanoxynitride und Titannitrid (TiOₓN_{y}, TiN, blau) zu nennen, wobei die niederen Titanoxide und -nitride in der Regel im Gemisch mit Titandioxid vorliegen.

Selbstverständlich können auch hier farblose hochbrechende Materialien, z.B. Metalloxide wie Zirkondioxid und insbesondere Titandioxid, verwendet werden, die mit selektiv absorbierenden Farbmitteln "eingefärbt" sind, was durch Einbau von Farbmitteln in die Metalloxidschicht, durch deren Dotierung mit selektiv absorbierenden Metallkatlonen oder durch Überziehen der Metalloxidschicht mit einem ein Farbmittel enthaltenden Film erfolgen kann (vgl. die nicht vorveröffentlichte DE-A-44 37 753).

Schließlich eignen sich als niedrigbrechende, nichtselektiv absorbierende Materialien mit hohen Absorptionskonstanten für die Beschichtung (B) insbesondere Metalle wie Aluminium.

Die Beschichtung (B) soll selbstverständlich nicht deckend, sondern für sichtbares Licht zumindest teilweise durchlässig (semitransparent) sein und ist daher in Abhängigkeit von den optischen Eigenschaften der ausgewählten Schichtmaterialien unterschiedlich dick.

Die Schichtdicke der Beschichtung (B) beträgt für nichtselektiv absorbierende hochbrechende Materiallen wie Metalle, schwarze Metalloxide und Sulfide im allgemeinen 1 bis 100 nm, wobei für stark absorbierende Metalle wie Molybdän und Chrom Schichtdicken von etwa 1 bis 25 nm, für schwächer absorbierende Materialien wie Magnetit Schichtdicken von etwa 10 bis 50 nm undfürmetallsulfidhaltige Materialien wie MoS₂-enthaltende Schichten Schichtdicken von 5 bis 20 nm bevorzugt sind.

Bei farbigen hochbrechenden Metalloxidbeschichtungen (B) beträgt die Schichtdicke üblicherweise 1 bis 500 nm, vorzugsweise 10 bis 150 nm.

Niedrigbrechende, aber stark absorbierende Aluminiumschichten (B) sind schließlich in der Regel 1 bis 25 nm, bevorzugt 5 bis 20 nm, dick.

Sind mehrere Schichtpakete (A) + (B) enthalten, so erniedrigt sich die Schichtdicke der Beschichtungen (B) üblicherweise um etwa 50 bis 75 %.

Durch die Beschichtung der hochbrechenden, (semi)transparenten Substrate mit der niedrigbrechenden Schicht (A) entsteht ein Pigment, das eine Serie von Interferenzfarben zeigt, die von den optischen Eigenschaften des Substrats (nicht absorbierend/absorbierend) bestimmt werden.

Nicht absorbierende (farblose) hochbrechende Substrate können bei entsprechenden Schichtdikken (etwa 40 bis 160 nm) schon von sich aus Interferenzfarben aufweisen. Durch eine Beschichtung mit (A) wird die Interferenzreihe an der vom Ausgangsmaterial bestimmten Stelle fortgesetzt, wobei die Interferenzfarbe gleichzeitig winkelabhängiger wird.

Beschichtet man beispielsweise silberne TiO₂₋belegte Glimmerplättchen oder silberne BiOCI-Plättchen mit Siliciumdioxid, so sind beim trockenen Pigmentpulver in der Aufsicht mit zunehmender SiO₂₋Schichtdicke mehrmals nacheinander die Interferenzfarben blau, grün, gold, rot zu beobachten. Ein in der Aufsicht blau reflektierendes Pigment zeigt z.B. in der Schrägsicht eine rote Farbe.

Die Interferenzfarben der mit (A) beschichteten nicht absorbierenden Substrate sind jedoch nur im trockenen Zustand, also beim Pigmentpulver, sichtbar und verschwinden im feuchten Zustand oder im Lack vollständig.

Durch zusätzliche Beschichtung mit einer nichtselektiv absorbierenden Schicht (B), z.B. mit Molybdän, werden die Interferenzfarben für jeden Farbton gleichermaßen verstärkt und bleiben auch im Lack sichtbar.

Bringt man eine selektiv absorbierende (farbige) Schicht (B) auf, werden insbesondere die Interferenzfarben verstärkt, die der Absorptionsfarbe von (B) nahe kommen, während davon abweichende Interferenzfarben geschwächt werden. So ist bei roten bis goldenen Interferenzfarben Eisen(III)oxid, bei grünen Interferenzfarben Chrom(III)oxid und bei blauen Interferenzfarben (insbesondere mit Ammoniak) reduziertes Titandioxid sehr gut geeignet.

Handelt es sich um absorbierende (semi) transparente Substrate, so muß zwischen nichtselektiv absorbierenden und selektiv absorbierenden Materialien unterschieden werden.

Nichtselektiv absorbierende Substrate erscheinen außerhalb des Glanzwinkels dunkel. Geeignete Substrate sind hier z.B. silberne TiO₂-belegte Glimmer, die bei 800°C mit Wasserstoff reduziert worden sind und die aufgrund der Bildung von reduzierten Titanoxiden bei fast unverändertem Silberglanz eine verringerte Lichtdurchlässigkeit aufweisen, und Glimmer mit rußdotierter TiO₂-Belegung.

Die nichtselektiv absorbierenden Substrate zeigen bei der Beschichtung mit der Schicht (A) an Luft kräftigere Interferenzfarben als die nicht absorbierenden Substrate.

Wie bei den transparenten Substraten werden auch bei ihnen die Interferenzfarben durch Beschichtung mit der Schicht (B) im Lack sichtbar, wobei selektiv absorbierende Schichten (B) wieder an die Interferenzfarben des mit (A) beschichteten Substrats angepaßt werden können.

Bei selektiv absorbierenden (farbigen) Substraten wie plättchenförmigen Eisenoxiden, mit Eisen (III)oxid beschichteten Glimmerplättchen und mit Ammoniak reduzierten (blauen) TiO₂-belegten Glimmerplättchen überlagert sich die Absorptionsfarbe des Substrats mit dem bei der Beschichtung mit der Schicht (A) entstehenden Interferenzsystem.

So zeigen mit (A) beschichtete Hämatitplättchen (α-Fe₂O_{3,}, rot) und Fe₂O₃-enthaltende Glimmer eine Serie von stark winkelabhängigen, sehr brillanten Interferenzfarben im grünstichig goldenen bis blaustichig roten Farbtonbereich, während grüne und blaue Farbtöne von dem in diesem Bereich absorbierenden Substrat geschwächt werden. Umgekehrt ergeben sich ausgehend von blauen reduzierten TiO₂-belegten Glimmerplättchen besonders brillante Interferenzfarben im blauen bis grünen Farbtonbereich.

Eine Verstärkung der Interferenzfarben kann wiederum durch Beschichtung mit der Schicht (B) erfolgen, wobei bei den selektiv absorbierenden Substraten selektiv absorbierende Schichten (B) bevorzugt sind, die, wie oben beschrieben, an die Interferenzfarben des Pigments (und damit an die Absorptionsfarbe des Substrats) angepaßt werden können.

Schließlich können die erfindungsgemäßen Glanzpigmente noch eine äußere Schicht (C) aufweisen, die insbesondere zum Schutz darunterliegender, im wesentlichen metallischer oder reduzierte (niederwertige) Metalloxide enthaltender Schichten (B) dient.

Die Schicht (C) kann aus niedrigbrechenden oder hochbrechenden Metalloxiden, die sowohl farblos als auch selektiv absorbierend sein können, aufgebaut sein. Geeignet sind z.B. Siliciumoxid, Siliciumoxidhydrat, Aluminiumoxid, Aluminiumoxidhydrat, Zinnoxid, Titandioxid, Zirkonoxid, Eisen(III)oxid und Chrom(III) oxid, wobei Siliciumoxid und Aluminiumoxid bevorzugt sind.

Die Schicht (C) kann auch eine durch Gasphasenpassivierung zu erhaltende, phosphat-, chromat- und/oder vanadathaltige oder auch phosphat- und Si0₂-haltige Schicht sein (EP-A-595 131 und die nicht vorveröffentlichte DE-A-44 14 079), die insbesondere auch den Einsatz der eine weitgehend metallische Schicht (B) aufweisenden, erfindungsgemäßen Glanzpigmente in Wasserbasislacken oder anderen wäßrigen Systemen ermöglicht.

Die Dicke der Schicht (C) beträgt im allgemeinen etwa 1 bis 400 nm, vorzugsweise 5 bis 250 nm.

Selbstverständlich kann auch die Schicht (C) zur Interferenz des Pigmentes beitragen und dabei die lnterferenzreihe an der durch das mit (A) und (B) beschichtete Substrat bestimmten Stelle fortsetzen. Dies ist beispielsweise der Fall, wenn Zirkon- oder Titanoxid als Schicht (C) aufgebracht werden. Besteht dagegen die Schicht (C) im wesentlichen aus Siliciumoxid, so wird sich diese Schicht im Anwendungsmedium (z.B. Lacken, Druckfarben oder Tinten), das einen ähnlichen Brechungsindex aufweist, koloristisch kaum bemerkbar machen.

Farbige Metalloxide wie Eisen- und Chromoxid werden schließlich die Interferenzfarbe des Mehrschichtsystems durch Beimischen ihrer Absorptionsfarbe modifizieren und mit zunehmender Schichtdicke auch überdecken.

Die erfindungsgemäßen Glanzpigmente zeichnen sich durch den gleichmäßigen, homogen und filmartigen Aufbau ihrer interferenzfähigen Beschichtung aus, welche die Substratplättchen allseitig umhüllt und nicht nur die Plättchenober- und -unterseite bedeckt.

Sie zeigen sehr kräftige und extrem winkelabhängige Interferenzfarben, die aufgrund der Lichtdurchlässigkeit der Substratteilchen nicht zu erwarten waren.

Im Gegensatz zu den bekannten stark reflektierenden goniochromatischen Glanzpigmenten auf metallischer Basis sind die erfindungsgemäßen Glanzpigmente noch für sichtbares Licht durchlässig, d.h., sie weisen in Auflicht und Durchlichtverschiedene (komplementäre) Interterenzfarben auf, zeichnen sich abertrotz ihrer Transparenz durch gutes Deckvermögen aus.

Außerdem zeigen sie im Lack nicht den "harten" metallischen Glanz, der für die metallischen Glanzpigmente üblich ist, sondern einen weicheren, scheinbar aus der Tiefe kommenden Glanz, weshalb sie in applizierter Form auch die Illusion räumlicher Tiefe hervorrufen.

Die erfindungsgemäßen Glanzpigmente werden bevorzugt durch mehrfache Beschichtung der Substratplättchen über Gasphasenzersetzung flüchtiger Metallverbindungen (CVD-Verfahren) oder naßchemisch über hydrolytische Zersetzung insbesondere organischer Metallverbindungen hergestellt.

Zur Erzeugung der Silicium- und/oder Aluminumoxidschichten (B) sind der naßchemische und der CVD-Herstellungsweg gleichermaßen geeignet.

Bei der naßchemischen Variante, die in der nicht vorveröffentlichten DE-A-44 05 492 beschrieben ist, werden organische Silicium- und/oder Aluminiumverbindungen, bei denen die organischen Reste über Sauerstoffatome an die Metalle gebunden sind, in Gegenwart der Substratteilchen und eines organischen Lösungsmittels, in welchem die Metallverbindungen löslich sind und das mit Wasser mischbar ist, hydrolysiert.

Die bevorzugte Ausführungsform besteht dabei in der Hydrolyse der Metallalkoholate (insbesondere Tetraethoxysilan und Aluminiumtriisopropanolat) In Gegenwart eines Alkohols (insbesondere Isopropanol) und von wäßrigem Ammoniak als Katalysator.

Verfahrenstechnisch geht man vorzugsweise so vor, daß man Substratteilchen, Isopropanol, Wasser und Ammoniak vorlegt, diese Mischung unter Rühren auf 40°C bis 80°C, insbesondere etwa 60°C bis 70°C, erhitzt und eine Lösung des Metallalkoholats in Isopropanol kontinuierlich zudosiert. Nach einer Nachrührzeit von meist etwa 1 bis 15 h kühlt man die Mischung auf Raumtemperatur ab und isoliert das beschichtete Pigment durch Abfiltrieren, Waschen und Trocknen.

Bei der CVD-Variante, die in der nicht vorveröffentlichten DE-A-44 37 752 beschrieben ist, werden Silane, die mindestens einen Alkanoyloxyrest enthalten, in der Gasphase mit Wasserdampf und, wenn die Silane auch Alkyl- oder Phenylreste aufweisen, Sauerstoff in Gegenwart bewegter Substratteilchen zersetzt.

Bevorzugte Silane weisen dabei Alkoxy- und Alkanoyloxyreste auf, besonders bevorzugt ist Di-tert.-butoxydiacetoxysilan.

Zur Durchführung der CVD-Variante empfiehlt sich wie allgemein für CVD-Verfahren die Verwendung eines Wirbelschichtreaktors, wie er beispielsweise in der EP-A 45 851 beschrieben ist. Die Substratteilchen werden im Reaktor unter Fluidisierung (Verwirbelung) mit einem inerten Wirbelgas wie Stickstoff auf die gewünschte Reaktionstemperatur (in der Regel 100 bis 600°C, bevorzugt 150 bis 300°C) erhitzt, Silan und Wasserdampf (sowie gegebenenfalls Sauerstoff) werden dann mit Hilfe inerter Trägergasströme (vorteilhaft Teilströmen des Wirbelgases) aus vorgeschalteten Verdampfergefäßen über getrennte Düsen eingeleitet, wobei die Silankonzentration zweckmäßigerweise bei≤ 5 Vol.-%, vorzugsweise ≤2 Vol.-%, bezogen auf die Gesamtgasmenge im Reaktor, gehalten wird. Die Wasserdampfmenge sollte mindestens der stöchiometrisch zur Hydrolyse des Silans erforderlichen Menge entsprechen, vorzuziehen ist jedoch die 10 bis 100fache Menge.

Die Erzeugung der Schichten (B) erfolgt vorzugsweise nach dem CVD-Verfahren, wobei in Abhängigkeit vom gewünschten Schichtmaterial unterschiedliche Reaktionsbedingungen einzustellen sind.

Wie aus der WO-A-93/12182 bekannt, werden metallische Schichten (B) bevorzugt durch Zersetzung von Metallcarbonylen wie Eisenpentacarbonyl, Chrom-, Molybdän-, Wolframhexacarbonyl, Nickeltetracarbonyl und/oder Dicobaltoctacarbonyl bei 70 bis 350°C unter inerten Bedingungen aufgebracht. Für die Zersetzung des besonders bevorzugten Mo(CO)₆ eignen sich dabei insbesondere Temperaturen von 200 bis 250°C.

Aluminiumschichten (B) können, wie in der älteren deutschen Patentanmeldung 19516181.5 beschrieben, durch inerte Gasphasenzersetzung von Aluminiumorganylen, insbesondere Aluminiumalkylen oder Alkylaminaddukten von Aluminiumhydriden, abgeschieden werden.

Als Aluminumalkyl eignen sich dabei neben Monoalkylaluminiumhydriden und -halogeniden vorzugsweise Dialkylaluminiumhydride und -halogenide und insbesondere Aluminiumtrialkyle, z.B. vor allem Triethyl- und Trimethylaluminium.

Verfahrenstechnisch geht man beim Aufbringen der Aluminiumschichten (B) zweckmäßigerweise so vor, daß man das Aluminiumalkyl in einem dem Beschichtungsreaktor vorgeschalteten, schrittweise auf ca. 100 bis 150°C erwärmten Verdampfergefäß als Lösung in einem schwerflüchtigen Kohlenwasserstoff wie Petroleum vorlegt, mit Hilfe eines durch diese Lösung geleiteten Inertgasstroms (z.B. Argon oder insbesondere Stickstoff) über eine vorzugsweise temperierte Düse in den Reaktor überführt und dort bei in der Regel 100 bis 500°C, vorzugsweise 150 bis 400°C, thermisch zersetzt, wobei die Gasmenge der flüchtigen Aluminiumverbindung im allgemeinen nicht mehr als 2 Vol.-%, bevorzugt nicht mehr als 1 Vol.-%, der Gesamtgasmenge im Reaktor betragen sollte.

Als Reaktor wird insbesondere der oben erwähnte Wirbelschichtreaktor bevorzugt, man kann jedoch auch einen Einhalsrundkolben aus Quarzglas verwenden, der über einen Motor gedreht wird, mit Gaszu- und -ableitungen in der Drehachse versehen ist und von einem zweischaligen Klappofen beheizt wird (Drehkugelofen). Im Prinzip läßt sich auch jeder beheizbare Mischer, der die Substratteilchen mittels entsprechender Einbauten schonend bewegt und eine Gaszu- und -ableitung gestattet, als Reaktor einsetzen. Für eine kontinuierliche Verfahrensführung in technischem Maßstab eignet sich z.B. auch ein Drehrohrofen, dem die Substratteilchen und die Aluminiumalkyl/inertgas-Mischung fortlaufend zugeführt werden.

Metallische Schichten (B) können schließlich auch naßchemisch durch Reduktion aus geeigneten Metallsalzlösungen aufgebrachtwerden. Auf diese Weise können vor allem edlere Metalle wie vor allem Silber, auch Kupfer, Gold, Cobalt, Nickel, Palladium und Platin abgeschieden werden. Wie in der EP-A-353 544 beschrieben, eignen sich hierfür eine Reihe von Reduktionsmitteln, insbesondere milde organische Reduktionsmittel, z.B. Zucker wie Glucose und Dextrose und auch Formaldehyd.

In der Regel werden jedoch die über die Gasphase aufgebrachten Metallschichten aufgrund ihrer höheren Qualität (feiner kristallin, filmartig) den naßchemisch aufgebrachten vorzuziehen sein, da sie meist brillantere und farbstärkere Glanzpigmente ergeben.

Die CVD-Abscheidung nichtselektiv absorbierender Schichten (B), die im wesentlichen aus niederen Metalloxiden (z.B. Fe₃O₄, VO₂, V₂O₃) bestehen, ist ebenfalls aus der WO-A-93/12182 bekannt. Die Metallcarbonyle wie Eisenpentacarbonyl oder Oxychloride wie Vanadiumoxychlorid werden hierbei mit Wasserdampf zersetzt. Wenn bei der Gasphasenzersetzung zunächst höhere Metalloxide wie V₂O₅ entstehen, müssen diese anschließend z.B. mit Wasserstoff oder Ammoniak zum gewünschten Oxid reduziert werden.

Wie in der EP-A-579 091 und der älteren deutschen Patentanmeldung 19515988.8 beschrieben, können nichtselektiv absorbierende metallsulfidhaltige Schichten (B) auf die mit (A) beschichteten Substratteilchen aufgebracht werden, indem man vorzugsweise durch Gasphasenzersetzung flüchtiger Metallverbindungen in Gegenwart eines Inertgases oder von Sauerstoff und/oder Wasserdampf zunächst eine Metall- bzw. Metalloxidschicht abscheidet und diese dann durch Umsetzung mit einer flüchtigen schwefelhaltigen Verbindung oder mit Schwefeldampf in die gewünschte metallsulfidhaltige Schicht (B) überführt oder die Schicht (B) direkt durch Gasphasenzersetzung flüchtiger Metallverbindungen in schwefelhaltiger Atmosphäre abscheidet.

Neben den in der EP-A-579 091 genannten schwefelhaltigen organischen Verbindungen sind als bevorzugte Schwefellieferanten insbesondere Schwefelwasserstoff und vor allem Schwefel selbst zu nennen.

Beim Einsatz von elementarem Schwefel geht man verfahrenstechnisch zweckmäßigerweise so vor, daß man feingemahlenes Schwefelpulver zusammen mit dem Substratmaterial in den Reaktor gibt, etwa 1 bis 4 h inertisiert und anschließend unter Ausschluß von Sauerstoff auf die Reaktionstemperatur (im allgemeinen 200 bis 500°C, bevorzugt 300 bis 500°C, besonders bevorzugt 400 bis 450°C) erhitzt.

Als Reaktoren eignen sich dabei die für die Beschichtung mit Aluminium genannten Reaktoren.

Eventuell vorhandene Schwefelreste lassen sich durch Sublimation im Inertgasstrom leicht entfernen. In der Regel wird dies jedoch nicht notwendig sein, da der Schwefel quantitativ (bis zu der für die Bildung des Metallsulfids stöchiometrisch erforderlichen Menge) umgesetzt wird und daher leicht in der dem gewünschten Sulfidgehalt der Schicht (B) entsprechenden Menge zugegeben werden kann. Bevorzugt wird so viel Schwefel eingesetzt, daß die bevorzugte metallische oder auch die oxidische Ausgangsschicht zumindest von einer zusammenhängenden, dichten Sulfidschicht bedeckt ist, die eine weitere Passivierung unnötig macht. Der innen (näher zum Substrat) liegende Bereich der Schicht (B) kann dabei nahezu kein Sulfid aufweisen und im wesentlichen nur aus dem jeweiligen Metall (bzw. Metalloxid) bestehen.

Auch zur Erzeugung selektiv absorbierender Schichten (B), die im wesentlichen aus farbigen Metalloxiden und/oder Metallnitriden bestehen, eignen sich insbesondere bereits beschriebene CVD-verfahren.

So sind die Abscheidung von α-Eisen(III)oxid, Chrom(III)oxid und Titan(III)oxid durch oxidative Zersetzung von Eisenpentacarbonyl und Chromhexacarbonyl bzw. hydrolytische Zersetzung von Titantetraisopropanolat oder Titantetrachlorid sowie anschließende Reduktion des gebildeten Titandioxids mit Wasserstoff oder mit Ammoniak sowie auch Ammoniak/Propan-Gemischen, wonach Ti₂O₃ (neben TiO₂) im Gemisch mit Titanoxynitriden und -nitriden (sowie auch eventuell Kohlenstoff) vorliegt, hinlänglich bekannt (EP-A-33 457, EP-A-338 428, ältere deutsche Patentanmeldungen 19511696.8 und 19511697.6).

Naßchemisch könnten α-Fe₂O₃- und Cr₂O₃₋Schichten durch hydrolytische Zersetzung von Eisen (III)salzen wie Eisen(III)chlorid und -sulfat bzw. Chrom (III)chlorid und anschließendes Überführen der gebildeten hydroxidhaftigen Schichten durch Tempern in die Oxidschichten aufgebracht werden. Ebenso könnte eine Ti₂O₃-Beschichtung durch Hydrolyse von TiCl₄ und anschließende Reduktion des gebildeten TiO₂ mit Wasserstoff oder Ammoniak erreicht werden.

Die Belegung mit selektiv absorbierendem γ-Fe₂O₃ (B) kann nach den in der DE-A-43 40 141 beschriebenen CVD-Verfahrensvarianten erfolgen, indem zunächst durch Zersetzung von Fe(CO)₅ in Gegenwart von Wasserdampf ein Magnetitfilm abgeschieden wird, der anschließend mit Luft zu γ-Fe₂O₃ oxidiert wird, oder zunächst durch oxidative Zersetzung von Fe(CO)₅ ein α-Fe₂O₃-Film abgeschieden wird, der mit Wasserstoff zu eisen(II)haltigen Produkten reduziert und anschließend mit Luft zu γ-Fe₂O₃ oxidiert wird.

Vanadium(V)oxidschichten (B) können schließlich durch Gasphasenzersetzung von Vanadiumoxychlorid mit Wasserdampf abgeschieden werden.

Zur Herstellung "eingefärbter" TiO₂-Schichten (B) sei auf die Angaben in der nicht vorveröffentlichten DE-A-44 37 753 verwiesen.

Äußere Schutzschichten (C), die im wesentlichen aus farblosen oder selektiv absorbierenden Metalloxiden bestehen, können nach den bereits beschriebenen Verfahren durch oxidative bzw. hydrolytische Gasphasenzersetzung der Metallcarbonyle bzw. der Metallalkoholate oder naßchemisch durch Hydrolyse organischer Metallverbindungen (Silicium, Aluminium) oder anorganischer Metallsalze erzeugt werden.

Phosphat-, chromat- und/oder vanadathaltige sowie phosphatund SiO₂-haltige äußere Schichten (C) können nach den in der EP-A-595 131 und in der nicht vorveröffentlichten DE-A-44 14 079 beschriebenen Passivierungsverfahren durch hydrolytische oder oxidative Gasphasenzersetzung von Oxidhalogeniden der Metalle (z.B. CrO₂Cl₂, VOCl₃), insbesondere von Phosphoroxyhalogeniden (z.B. POCl₃), Phosphor- und Phosphorigsäureestern (z.B. Di- und Trimethyl- und -ethylphosphit) und von Aminogruppen enthaltenden Siliciumorganylen (z.B. 3-Aminopropyltriethoxy- und -trimethoxysilan) aufgebracht werden.

Glanzpigmente, die in wäßrigen Systemen besonders stabil sind, werden bei kombinierter Zersetzung der Phosphor- und Siliciumverbindungen erhalten.

Die erfindungsgemäßen Glanzpigmente eignen sich vorteilhaft für viele Zwecke, wie zur Einfärbung von Kunststoffen, Gläsern, keramischen Produkten, Zubereitungen der dekorativen Kosmetik und besonders von Lacken, insbesondere auch Automobillacken, Tinten und Druckfarben, vor allem Sicherheitsdruckfarben. Bei der Applikation im Druck sind alle bekannten Druckverfahren, z.B. Siebdruck, Tiefdruck, Bronzierdruck, Flexodruck und Offsetdruck, geeignet.

Für diese Anwendungszwecke lassen sich die erfindungsgemäßen Pigmente auch vorteilhaft in Abmischung mit transparenten und deckenden Weiß-, Bunt- und Schwarzpigmenten sowie auch herkömmlichen transparenten, bunten und schwarzen Glanzpigmenten auf der Basis von metalloxidbeschichteten Glimmer- und Metallpigmenten, plättchenförmigen Eisenoxiden, Graphit, Molybdänsulfid und plättchenförmigen organischen Pigmenten verwenden.

### Beispiele

### Herstellung und Anwendung von erfindungsgemäßen Glanzpigmenten

Bei der Einarbeitung der Pigmente in Lack wurden jeweils 0,4 g Pigment in 3,6 g eines Polyester-Mischlackes mit 21 Gew.-% Feststoffanteil eingerührt und 2 min im Red Devil dispergiert. Mit einer Rakel (160 µm Naßfilmdicke) wurden auf schwarzweißem Karton Abzüge der pigmentierten Lacke angefertigt.

### Beispiel 1

100 g eines silberfarbenen TiO₂-beschichteten Glimmerpigments (Iriodin® 103 Rutil Sterling Silber; Merck) wurden in einem Drehkugelofen 1 h durch Überleiten von 50 l/h Stickstoff inertisiert. Nach Erhitzen auf 600°C wurde 2 h ein Wasserstoffstrom von 20 l/h eingeleitet. Nach erfolgter Reduktion wurde unter emeutem Spülen mit Stickstoff auf Raumtemperatur abgekühlt.

Das ursprünglich weiße Pigment zeigte nach der Reduktion eine silberne Körperfarbe und besseres Deckvermögen.

In einem mit Rückflußkühler und Rührapparatur versehenen Rundkolben wurde das reduzierte Glimmerpigment in 800 ml Isopropanol aufgeschlämmt. Nach Zugabe von 300 ml Wasser und 30 ml einer 25 gew.%igen wäßrigen Ammoniaklösung wurde die Suspension unter kräftigem Rühren auf 60°C erhitzt. Gleichzeitig wurde mit der Zudosierung eines Gemisches von 200 ml Isopropanol und 400 g Tetraethoxysilan begonnen (Dosiergeschwindigkeit 100 ml/h, 6 h). Nach einer Nachrührzeit von 2 h und Abkühlen der Suspension wurde das Produkt abfiltriert, gründlich mit Isopropanol gewaschen und bei 80°C getrocknet.

Das getrocknete SiO₂-beschichtete Pigment zeigte an der Luft in Aufsicht eine schwachblaue Interferenzfarbe, die in Schrägsicht nach Schwachrot abkippte.

Anschließend wurde das SiO₂-beschichtete Glimmerpigment (210 g) in einem Wirbelschichtreaktor unter Fluidisierung mit insgesamt 600 l/h Stickstoff auf 220°C erhitzt. Aus einer auf 60°C erwärmten Vorlage wurden mit einem zusätzlich Stickstoffstrom von 400 l/h 32,3 g Molybdänhexacarbonyl in 8 h in den Reaktor überführt, wo sie zu Molybdän und Kohlenmonoxid zersetzt wurden. Nach beendeter Molybdänabscheidung wurde den Wirbelgasen beim Abkühlen des Reaktors zur Passivierung der Molybdänoberfläche etwas Luft zugesetzt. Das mit Molybdän beschichtete Pigment zeigte im Lack in der Aufsicht eine kräftige, grünstichig blaue Interferenzfarbe, die in der Schrägsicht über reines Blau nach Violett abkippte.

100 g des Mo-beschichteten Pigments wurden dann mit 2,5 g feingemahlenem Schwefelpulver gemischt, im Drehkugelofen zunächst durch einstündiges Überleiten von 30 l/h Stickstoff inertisiert und anschlie-βend unter einem Stickstoffstrom von 5 Uh in etwa 30 min auf 500°C erhitzt. Nach 2 h wurde unter Stickstoff auf Raumtemperatur abgekühlt.

Das erhaltene Pigment hatte einen Titangehalt von 7,5 Gew.-%, einen Siliciumgehalt von 30,5 Gew.-%, einen Molybdängehalt von 3,6 Gew.-% und einen Schwefelgehalt von 0,96 Gew.-%. Bei Applikation zeigte es in der Aufsicht eine kräftige, blaugrüne Interferenzfarbe, die bei zunehmender Schrägsicht über Violett nach Rot abkippte.

### Beispiel 2

100 g des silberfarbenen TiO₂-beschichteten Glimmerpigments wurden analog Beispiel 1, jedoch bei 800°C mit Wasserstoff reduziert.

Das Pigment zeigte nach der Reduktion ebenfalls eine silberne Körperfarbe und ein gegenüber dem Pigment aus Beispiel 1 verbessertes Deckvermögen.

100 g des reduzierten Pigments wurden analog Beispiel 1 mit SiO₂ beschichtet, indem sie in 100 ml Isopropanol aufgeschlämmt, zunächst mit 400 ml Wasser und 40 ml 25 gew.-%igem Ammoniak und dann in 9 h mit einem Gemisch von 300 ml Isopropanol und 600 g Tetraethoxysilan versetzt wurden. Die Nachrührzeit betrug 14 h.

Das getrocknete SiO₂-beschichtete Pigment (268 g) zeigte an der Luft in Aufsicht einen blauvioletten Schimmer, der bei Schrägsicht in einen roten Schimmer überging.

Anschließend wurden 185 g des SiO₂-beschichteten Pigments analog Beispiel 1 unter Verwendung von 27,5 g Mo(CO)₆ in 6 h mit Molybdän beschichtet.

Das Mo-beschichtete Pigment zeigte im Lack in der Aufsicht eine kräftige, blaue Interferenzfarbe, die in der Schrägsicht nach Violett abkippte.

100 g des Mo-beschichteten Pigments wurden dann analog Beispiel 1 mit 4,5 g Schwefelpulver umgesetzt.

Das erhaltene Pigment hatte einen Ti-Gehalt von 6,0 Gew.-%, einen Si-Gehalt von 31 Gew.-%, einen Mo-Gehalt von 3,3 Gew.-% und einen S-Gehalt von 1,7 Gew.-%. Bei Applikation zeigte es in der Aufsicht eine kräftige, rotstichig blaue Interferenzfarbe, die bei zunehmender Schrägsicht über Rot nach Gold abkippte.

### Beispiel 3

150 g des silberfarbenen TiO₂-beschichteten Glimmerpigments wurden analog Beispiel 1 mit SiO₂ beschichtet, indem sie in 150 ml Isopropanol aufgeschlämmt, zunächst mit 500 ml Wasser und 50 ml 25 gew.-%igem Ammoniak und dann in 7 h mit einem Gemisch von 375 ml Isopropanol und 750 g Tetraethoxysilan versetzt wurden (Dosiergeschwindigkeit 160 ml/h). Die Nachrührzeit betrug 1 h.

Das getrocknete SiO₂-beschichtete Pigment (352 g) behielt seine weiße Körperfarbe und zeigte an der Luft in Schrägsicht nur über schwarzem Untergrund eine schwache, rote Interferenzfarbe.

Anschließend wurden 310 g des SiO₂-beschichteten Pigments analog Beispiel 1 unter Verwendung von 49,8 g Mo(CO)₆ in 15 h mit Molybdän beschichtet.

Das Mo-beschichtete Pigment zeigte im Lack in der Aufsicht eine kräftige, rote Interferenzfarbe, die bei zunehmender Schrägsicht über Rotgold nach Grüngold abkippte.

100 g des Mo-beschichteten Pigments wurden dann analog Beispiel 1 mit Schwefelpulver umgesetzt.

Das erhaltene Pigment hatte einen Ti-Gehalt von 8,4 Gew.-%, einen Si-Gehalt von 27,4 Gew.-%, einen Mo-Gehalt von 4,6 Gew.-% und einen S-Gehalt von 1,2 Gew.-%. Bei Applikation zeigte es in der Aufsicht eine kräftige, blaustichig rote Interferenzfarbe, die bei zunehmender Schrägsicht über Rot nach Gold abkippte.

### Beispiel 4

150 g eines blausilbernen, mit Ammoniak reduzierten, TiO₂-beschichteten Glimmerpigments (Paliocrom® Blausilber L 6000; BASF) wurden analog Beispiel 1 mit SiO₂ beschichtet, indem sie in 1500 ml Isopropanol aufgeschlämmt, zunächst mit 500 ml Wasser und 50 ml 25 gew.-%igem Ammoniak und dann in 7 h mit einem Gemisch von 300 ml Isopropanol und 600 g Tetraethoxysilan versetzt wurden. Die Nachrührzeit betrug 14 h.

Das getrocknete SiO₂-beschichtete Pigment (312 g) zeigte an der Luft in Aufsicht eine intensiv blaue lnterferenzfarbe, die in Schrägsicht nach Violett abkippte.

Anschließend wurden 300 g des SiO₂-beschichteten Pigments analog Beispiel 1 unter Verwendung von 30 g Mo(CO)₆ in 7 h mit Molybdän beschichtet.

Das erhaltene Pigment hatte einen Ti-Gehalt von 7,7 Gew.%, einen Si-Gehaft von 29,6 Gew.% und einen Mo-Gehalt von 2,6 Gew.%. Bei Applikation zeigte es in der Aufsicht eine kräftige, blaue Interferenzfarbe, die in der Schrägsicht nach Violett abkippte.

### Beispiel 5

2 g des SiO₂-beschichteten Pigments aus Beispiel 2 wurden in 100 ml Wasser aufgeschlämmt. Nach Zugabe von 1 g Dextrose wurde der pH-Wert der Suspension mit 2 gew.-%igem Ammoniak auf 9 eingestellt. Nach Zugabe einer Lösung von 0,2 g Silbemitrat in 50 ml Wasser wurde die Suspension auf 40°C erwärmt und 2 h bei dieser Temperatur und weitere 15 h bei Raumtemperatur gerührt. Das Produkt wurde abfiltriert, zunächst mit Wasser und dann mit Aceton gewaschen und bei Raumtemperatur getrocknet.

Das erhaltene Pigment hatte einen Ti-Gehalt von 7,4 Gew.-%, einen Si-Gehalt von 34,4 Gew.% und einen Silbergehalt von 6,6 Gew.-%. Bei Applikation in Lack zeigte es in der Aufsicht eine graublaue Farbe, die sich bel zunehmender Schrägsicht über Rot nach Grün verschob.

### Beispiel 6

150 g eines kupferfarbenen, mit Aluminium und Mangan dotierten, plättchenförmigen α-Eisen(III) oxidpigments (2,2 Gew.-% Aluminium, 0,3 Gew.-% Mangan, jeweils bezogen auf das Gesamtpigment; mittlerer Teilchendurchmesser 18 µm; analog Beispiel 1 der EP-A-265 280, jedoch in einem 3,5-I-Autoklav mit 1 Ofachem Ansatz hergestellt) wurden analog Beispiel 1 mit SiO₂ beschichtet, indem sie in 1200 ml Isopropanol aufgeschlämmt, zunächst mit 500 ml Wasser und 50 ml 25 gew.-%igem Ammoniak und dann in 5 h mit 500 g Tetraethoxysilan versetzt wurden. Die Nachrührzeit betrug 2h.

Das getrocknete SiO₂-beschichtete Pigment (280 g) zeigte an der Luft in Aufsicht eine rote lnterferenzfarbe, die in Schrägsicht nach Grüngold abkippte.

Anschließend wurden 120 g des SiO₂-beschichteten Pigments in einem Wirbelschichtreaktorunter Fluidisierung mit 400 l/h Stickstoff auf 190°C erhitzt. Über zwei weitere seitliche Düsen wurden zusätzlich 300 l/h Stickstoff, der durch Überleiten über eine auf 40°C temperierte Wasservorlage mit Wasserdampf beladen worden war, sowie 200 l/h Luft zugeführt. Aus einer auf Raumtemperatur gehaltenen Vorlage wurden mit weiteren 300 l/h Stickstoff 120 g Eisenpentacarbonyl in 12 h in den Reaktor überführt und dort zu α-Fe₂O₃ zersetzt.

Das erhaltene Pigment hatte einen Si-Gehalt von 18,4 Gew.-% und einen Gesamteisengehalt von 37,5 Gew.-%. Bei Applikation zeigte es in der Aufsicht eine grünstichige Interferenzfarbe, die mit zunehmender Schrägsicht über Blau nach Rot abkippte.

## Patentansprüche

1. Goniochromatische Glanzpigmente auf der Basis von mehrfach beschichteten, hochbrechenden, für sichtbares Licht zumindesc teilweise durchlässigen, nichtmetallischen, plättchenförmigen Substraten, die mindestens ein Schichtpaket aus
A) einer farblosen Beschichtung mit einem Brechungsindex n ≤ 1,8 und
B) einer reflektierenden, selektiv oder nichtselektiv absorbierenden, für sichtbares Licht zumindest teilweise durchlässigen Beschichtung
sowie gewünschtenfalls zusätzlich
C) eine äußere Schutzschicht
aufweisen,
wobei die Schichtdicke der Schicht (A) 100 bis 600 nm beträgt, wenn die Glanzpigmente ein Schichtpaket (A) + (B) aufweisen, und 50 bis 300 rim beträgt, wenn die Glanzpigmente mehrere Schichtpakete (A) + (B) aufweisen.

2. Glanzpigmente nach Anspruch 1, bei denen das plättchenförmige Substrat einen Brechungsindex n ≥ 2,0 aufweist.

3. Glanzpigmente nach Anspruch 1 oder 2, bei denen das plättchenförmige Substrat im wesentlichen aus silikatischen Plättchen, die mit einer hochbrechenden, für sichtbares Licht zumindest teilweise durchlässigen Schicht belegt sind, oder plättchenförmigen Eisenoxiden besteht.

4. Glanzpigmente nach den Ansprüchen 1 bis 3, bei denen das plättchenförmige Substrat im wesentlichen aus mit hochbrechenden Metalloxiden beschichteten GlimmerpLättchen besteht.

5. Glanzpigmente nach den Ansprüchen 1 bis 4, bei denen die Beschichtung (A) im wesentlichen aus niedrigbrechenden Metalloxiden und/oder Magnesiumfluarid besteht.

6. Glanzpigmente nach den Ansprüchen 1 bis 5, bei denen die Beschichtung (A) im wesentlichen aus Siliciumoxid, Silicumoxidhydrat, Aluminiumoxid und/oder Aluminiumoxidhydrat, besteht.

7. Glanzpigmente nach den Ansprüchen 1 bis 6, bei denen die Beschichtung (B) im wesentlichen aus Metallen, Metalloxiden, . Metallsulfiden und/oder Metallnitriden besteht.

8. Glanzpigmente nach den Ansprüchen 1 bis 7, bei denen die Schutzschicht (C) im wesentlichen aus farblosen oder selektiv absorbierenden Metalloxiden besteht und/oder phosphat-, chromat- und/oder vanadathaltig ist,

9. Glanzpigmente nach den Ansprüchen 1 bis 8, die nur ein Schichtpaket (A) + (B) enthalten.

10. Verwendung von Glanzpigmenten gemäß den Ansprüchen 1 bis 9 zur Einfärbung von Lacken, Druckfarben, Tinten, Kunststoffen, Gläsern, keramischen Produkten und Zubereitungen der dekorativen Kosmetik.

## Claims

1. Goniochromatic luster pigments based on multiply coated, high refractive, nonmetallic, platelet-shaped substrates which are at least partially transparent to visible light, comprising at least one layer packet of
A) a colorless coating having a refractive index n ≤ 1.8 and
B) a reflecting, selectively or nonselectively absorbing coating which is at least partially transparent to visible light,
and also, if desired, additionally
C) an outer protective layer,
wherein the layer thickness of the layer (A) is from 100 to 600 nm if the luster pigments have one layer packet (A) + (B) and is from 50 to 300 nm if the luster pigments have a plurality of layer packets (A) + (B).

2. Luster pigments according to claim 1 wherein the platelet-shaped substrate has a refractive index n ≥ 2.0.

3. Luster pigments according to claim 1 or 2 wherein the platelet-shaped substrate consists essentially of silicatic platelets coated with a high refractive layer which is at least partially transparent to visible light, or of platelet-shaped iron oxides.

4. Luster pigments according to any of claims 1 to 3 wherein the platelet-shaped substrate consists essentially of mica platelets coated with high refractive metal oxides.

5. Luster pigments according to any of claims 1 to 4 wherein said coating (A) consists essentially of low refractive metal oxides and/or magnesium fluoride.

6. Luster pigments according to any of claims 1 to 5 wherein said coating (A) consists essentially of silicon oxide, silicon oxide hydrate, aluminum oxide and/or aluminum oxide hydrate.

7. Luster pigments according to any of claims 1 to 6 wherein said coating (B) consists essentially of metals, metal oxides, metal sulfides and/or metal nitrides.

8. Luster pigments according to any of claims 1 to 7 wherein said protective layer (C) consists essentially of colorless or selectively absorbing metal oxides and/or is phosphate-, chromate- and/or vanadate-containing.

9. Luster pigments according to any of claims 1 to 8 comprising only one layer packet (A) + (B).

10. The use of luster pigments according to any of claims 1 to 9 for coloring paints, inks, including printing inks, plastics, glasses, ceramic products and decorative cosmetic preparations.

## Revendications

1. Pigments brillants goniochromatiques à base de substrats en forme de plaquette non métalliques, présentant un recouvrement à plusieurs couches, qui sont fortement réfractants et, au moins partiellement, translucides à la lumière visible, comprenant au moins un ensemble de couches constitué de
A) un revêtement incolore présentant un indice de réfraction n ≤ 1,8 et
B) un revêtement réflecteur qui est apte à une absorption sélective ou non sélective et qui est, au moins partiellement, translucide à la lumière visible,
ainsi qu'éventuellement en plus
C) une couche extérieure protectrice, l'épaisseur de la couche (A) étant comprise entre 100 et 600 nm lorsque pigments brillants présentent un ensemble de couches (A)+(B) et étant comprise entre 50 et 300 nm lorsque les pigments brillants présentent plusieurs ensembles de couche (A) + (B) .

2. Pigments brillants selon la revendication 1, **caractérisés en ce que** le substrat en forme de plaquette présente un indice de réfraction n ≥ 2,0.

3. Pigments brillants selon la revendication 1 ou 2, **caractérisés en ce que** le substrat en forme de plaquette est constitué pour l'essentiel de plaquettes constituées de silicate qui sont recouvertes d'une couche fortement réfractive et, au moins partiellement, translucide à la lumière visible, ou bien d'oxydes de fer sous forme de plaquettes.

4. Pigments brillants selon les revendications 1 à 3, **caractérisés en ce que** le substrat en forme de plaquette est constitué pour l'essentiel de plaquettes de mica recouvertes d'oxydes métalliques fortement réfractifs.

5. Pigments brillants selon les revendications 1 à 4, **caractérisés en ce que** le revêtement (A) est constitué pour l'essentiel du fluorure de magnésium et/ou d'oxydes métalliques à réfraction faible.

6. Pigments brillants selon les revendications 1 à 5, **caractérisés en ce que** le revêtement (A) est constitué pour l'essentiel d'oxyde de silicium, d'hydrate d'oxyde de silicium, d'oxyde d'aluminium et/ou d'hydrate d'oxyde d'aluminium.

7. Pigments brillants selon les revendications 1 à 6, **caractérisés en ce que** le revêtement (B) est constitué pour l'essentiel de métaux, d'oxydes métalliques, de sulfures métalliques et/ou de nitrures métalliques.

8. Pigments brillants selon les revendications 1 à 7, **caractérisés en ce que** la couche protectrice (C) est constituée pour l'essentiel d'oxydes métalliques à absorption sélective ou incolores, et/ou **en ce qu'**elle contient un phosphate, un chromate et/ou un vanadate.

9. Pigments brillants selon les revendications 1 à 8, contenant seulement un ensemble de couches (A) + (B).

10. Mise en oeuvre de pigments brillants selon les revendications 1 à 9 pour la coloration de laques, d'encres d'impression, d'encres, de matières synthétiques, de verres, de produits céramiques et de préparations destinées à l'esthétique décorative.
